# EUROPEAN PATENT APPLICATION

(11) **EP 4 464 269 A1**
(43) Date of publication of application: **20.11.2024**
(21) Application number: 23173787.5
(22) Date of filing: 16.05.2023
(51) Int. Cl.: A61B 17/70, A61B 17/84, A61B 17/86

(54) **A BOLT FOR THE FIXATION OF BONES**

(71) Applicant: Sota Orthopaedics Limited, Malahide K36EY13 Dublin (IE)
(72) Inventor: THORNES, Brian, Dublin, K36 EY13 (IE)
(74) Representative: FRKelly

(57) **Abstract**

This invention relates to a bolt for the fixation of bones. The bolt finds utility as a bolt for fixation of bones such as vertebra of the spine, although it may be used in any suitable bone.

## Description

### Field of the invention

This invention relates to a bolt for the fixation of bones. The bolt finds utility as a bolt for fixation of bones such as vertebra of the spine, although it may be used in any suitable bone.

### Background to the invention

Each vertebra of the vertebral column comprises an anterior-facing vertebral body and a posteriorfacing vertebral arch, which together define the vertebral foramen, a closed aperture enclosing the spinal canal, through which the spinal cord passes. The vertebral arch comprises two pedicles, two laminae, and seven processes. The pedicles connect the vertebral body and the vertebral arch.

Pedicle screw fixation is commonly used in spinal instrumentation surgeries to connect rods to vertebrae in order to correct spine alignment, stabilize vertebrae, and reach an arthrodesis. Indeed, trans-pedicle screw fixation is the gold standard of vertebral fixation, such that pedicle screw fixation is commonplace for securing anchorage in thoraco-lumbar spinal surgery in order to treat a variety of conditions, including degenerative, deformity, tumour and trauma applications.

In pedicle screw fixation, pedicle screws are placed from the posterior aspect of the spine. After surgical exposure, the anatomical landmarks are identified. The posterior cortical crest of bone is removed, for example with a rongeur or burr, to expose the underlying cancellous bone. The entry point is prepared, preferably with an awl. Using a curved blunt probe, a pathway into the pedicle is created. The probe should follow a path of least resistance without violating the pedicle walls. If resistance is felt, the entry point and trajectory should be re-evaluated. At any stage, correct position can be confirmed, for example with fluoroscopy. Other instruments, such as a pedicle feeler or drilling tool (such as PediGuard^{®} by SpineGuard) can also be used to assist positioning and/or trajectory. For increased bone purchase, bone taps can be used to prepare the pedicle canal.

The pedicle screw head has a large U-shaped component to engage with the posterior spinal rods. This pedicle screw head can either be monoaxial, polyaxial or uniplanar, to facilitate the ease of, and appropriate connection with, the posterior metal rods. The pedicle screws are inserted and gain anchorage in the pedicle in the subcortical (cancellous) bone and to a lesser extent in the vertebral body in the cancellous bone.

A pedicle screw relies mostly on a short segment of anchorage between screw and subcortical (cancellous) bone on the inner aspect of the pedicle. Extreme care must be taken to avoid breaching the cortical wall of the pedicle, so as not to enter the spinal canal and injure the spinal cord and/or nerve roots. If the cortical bone is breached, then subsequent anchorage with a pedicle screw is greatly weakened. Anchorage may also be difficult in revision surgery and in patients with osteoporotic or pathological bone. Loss of screw anchorage (pull-out) or screw breakage typically occurs from micro-movement in the flexion-extension plane, with possibly small additional rotational stresses.

To be effective, a pedicle screw must withstand intraoperative loading and physiological forces due to daily postoperative activities. The pedicle screw needs to resist flexion-extension forces and to a lesser extent, lateral flexion forces and rotational torque forces. The cross section of a pedicle is oblong, longer in superior-inferior dimension than medio-lateral dimension, but pedicle screws by their nature are circular in cross section and therefore cannot maximise the anatomical resources of bone for fixation.

It is an object of the present invention to provide an improved device for vertebral fixation, which provides a means for reducing the occurrence of screw pull-out and screw breakage, and which can resist flexion-extension forces, lateral flexion forces and/or rotational torque forces.

### Summary of the invention

According to a first aspect of the present invention there is provided a bolt for the fixation of bones, the bolt comprising a body having an outer surface, wherein at least a part of the outer surface of the body comprises at least one threaded portion.

According to a second aspect of the present invention there is provided a bolt for the fixation of bones, the bolt comprising a body having an expandable section.

According to a third aspect of the present invention there is provided a bolt for the fixation of bones, the bolt comprising a body having an expandable section and an outer surface, wherein at least a part of the outer surface of the body comprises at least one threaded portion.

Optionally, the expandable section is adjacent the outer surface of the body.

Optionally, expandable section and the outer surface of the body are contiguous.

Optionally, expandable section and the outer surface of the body are conterminous.

Optionally or additionally, the expandable section and the outer surface of the body are integral.

Optionally, at least a part of the expandable section comprises at least one threaded portion. Further optionally, at least a part of the outer surface of the expandable section comprises at least one threaded portion.

Alternatively, at least a part of the expandable section does not comprise a threaded portion. Further alternatively, at least a part of the outer surface of the expandable section does not comprise a threaded portion. Still further alternatively, at least a part of the expandable section excludes a threaded portion. Still further alternatively, at least a part of the outer surface of the expandable section excludes a threaded portion.

Preferably, the expandable section excludes a threaded portion. Further preferably, the outer surface of the expandable section excludes a threaded portion.

Optionally, the body has a proximal end and a distal end.

Optionally, the expandable section is adjacent or at the distal end.

Optionally or additionally, the outer surface of the body is adjacent or at the proximal end.

By the term "lead" is meant a distance along an axis, optionally a longitudinal axis, of the at least one threaded portion that is covered by a rotation, optionally a 360° rotation, of the at least one threaded portion.

By the term "pitch" is meant a distance along an axis, optionally a longitudinal axis, of the at least one threaded portion from a first point of a thread to a corresponding point on an adjacent thread.

Optionally, the lead of the threaded portion is greater than the pitch of the at least one threaded portion.

Optionally, the lead of the threaded portion is at least two times, optionally at least three times, optionally at least four times greater than the pitch of the at least one threaded portion.

Preferably, the lead of the threaded portion is two times greater than the pitch of the at least one threaded portion.

Optionally, the at least one threaded portion comprises a thread having at least one lead.

Optionally, the at least one threaded portion comprises a single-lead thread.

Optionally, the at least one threaded portion comprises a thread having at least two, optionally at least three, optionally at least four leads.

Optionally, the at least one threaded portion comprises a double-, optionally a triple-, optionally a quadruple-lead thread.

Preferably, the at least one threaded portion comprises a thread having two leads.

Preferably, the at least one threaded portion comprises a double-lead thread.

Optionally, the at least one threaded portion comprises a thread having at least one start.

Optionally, the at least one threaded portion comprises a single-start thread.

Optionally, the at least one threaded portion comprises a thread having at least two, optionally at least three, optionally at least four starts.

Optionally, the at least one threaded portion comprises a double-, optionally a triple-, optionally a quadruple-start thread.

Preferably, the at least one threaded portion comprises a thread having two starts.

Preferably, the at least one threaded portion comprises a double-start thread.

Optionally, the at least one threaded portion comprises at least one thread.

Optionally, the at least one threaded portion comprises at least two, optionally at least three, optionally at least four threads.

Optionally, the at least one threaded portion comprises at least one helical thread.

Optionally, the at least one threaded portion comprises at least two, optionally at least three, optionally at least four helical threads.

Preferably, the at least one threaded portion comprises two threads.

Preferably, the at least one threaded portion comprises two helical threads.

Optionally, the expandable section has respective ends, and is operable between a contracted position and an expanded position.

Optionally, the bolt further comprises expanding means.

According to a fourth aspect of the present invention there is provided a bolt for the fixation of bones, the bolt comprising: (a) a body having an expandable section and an outer surface, wherein at least a part of the outer surface of the body comprises at least one threaded portion; and (b) expanding means.

Optionally, the expanding means is in operable association with the expandable section to displace the expandable section between the contracted position and the expanded position by applying force to at least one respective end of the expandable section, such that the at least one respective end of the expandable section is advanced toward the opposing respective end.

Optionally, the expanding means is in operable association with the expandable section to displace the expandable section between the contracted position and the expanded position by applying force to the respective ends of the expandable section, such that each of the respective ends of the expandable section are advanced toward the opposing respective end.

Optionally, the expanding means displaces the expandable section between the contracted position and the expanded position by simultaneously applying force to the respective ends of the expandable section, such that each of the respective ends of the expandable section are advanced toward the opposing respective end.

Optionally, the expanding means is in operable association with the expandable section to displace the expandable section between the contracted position and the expanded position by applying force to the respective ends of the expandable section, such that each of the respective ends of the expandable section are independently advanced toward the opposing respective end.

Further optionally, the expanding means is in operable association with the expandable section, to displace the expandable section between the contracted position and the expanded position by simultaneously applying force to the respective ends of the expandable section, such that each of the respective ends of the expandable section are independently advanced toward the opposing respective end.

Optionally, the expandable section is reversibly expandable. More optionally, the expandable section is reversibly expandable under mechanical pressure.

Optionally, the expandable section is collapsible, optionally along a longitudinal axis. Further optionally, the expandable section is radially inwardly collapsible, optionally along a longitudinal axis.

Optionally, the expandable section comprises at least two expandable members that extend from a longitudinal axis of the bolt under mechanical pressure. More optionally, the expandable members extend radially from a longitudinal axis of the bolt under mechanical pressure.

Optionally, each of the expandable members comprises a deformable arm.

Optionally, at least one point of folding is provided along each deformable arm.

Optionally, the or each point of folding comprises a point of weakness, a hinge mechanism, or any such mechanism that will facilitate the folding of the deformable arm at a desired location.

Optionally, the expanding means comprises a connecting means and at least one body mountable to the connecting means.

Optionally, the connecting means comprise a shaft. Preferably, the connecting means comprise a shaft.

Optionally, the connecting means comprise a shaft adapted to allow reciprocal movement of the at least one mountable body relative to the connecting means.

Optionally, the at least one mountable body is threadably mountable to the connecting means. Further optionally, a first mountable body is threadably mountable to the connecting means and arranged for displacement in response to rotation of the connecting means to apply mechanical pressure to at least one respective end of the expandable section. The connecting means can be arranged to simultaneously and independently apply mechanical pressure to at least one respective end of the expandable section. The at least one mountable body can be displaced in response to rotation of the connecting means relative to the at least one mountable body.

Optionally, the connecting means comprises at least one screw thread. Preferably, the connecting means comprises a screw thread.

Optionally, the at least one mountable body is mountable to the at least one screw thread of the connecting means. Preferably, the at least one mountable body is mountable to the screw thread of the connecting means.

Optionally, the at least one mountable body is engagable with at least one of the respective ends of the expandable section. Further optionally, the at least one mountable body is irreversibly engagable with at least one of the respective ends of the expandable section.

Optionally, the at least one mountable body defines a portion of the expandable section. Further optionally, the at least one mountable body comprises a screw thread located on a portion of the expandable section.

Optionally, the at least one mountable body comprises a screw thread located on at least part of the inner surface of the expandable section.

Preferably, the at least one mountable body comprises a screw thread located on at least part of the inner surface of the expandable section.

Optionally, the screw thread located on at least part of the inner surface of the expandable section is mountable to the at least one screw thread of the connecting means. Preferably, the screw thread located on at least part of the inner surface of the expandable section is mountable to the screw thread of the connecting means.

Optionally or additionally, the at least one mountable body is fixedly mounted to the connecting means. Further optionally, a second mountable body is fixedly mounted to the connecting means to apply mechanical pressure to at least one respective end of the expandable section. The connecting means can be arranged to simultaneously and independently apply mechanical pressure to at least one respective end of the expandable section.

Optionally, the at least one mountable body is engagable with at least one of the respective ends of the expandable section. Further optionally, the at least one mountable body is reversibly engagable with at least one of the respective ends of the expandable section.

Optionally, the at least one mountable body defines a portion of the connecting means. Further optionally, the at least one mountable body comprises a stop located on a portion of the connecting means.

Optionally, the expanding means comprises a connecting means and at least two mountable bodies. Further optionally, the expanding means comprises a connecting means and two mountable bodies. Still further optionally, the expanding means comprises a connecting means and first and second mountable bodies.

Optionally, a first mountable body is threadably mountable to the connecting means. Further optionally, the first mountable body is threadably mountable to the connecting means and arranged for displacement in response to rotation of the connecting means to apply mechanical pressure to at least one respective end of the expandable section. The first mountable body can be displaced in response to rotation of the connecting means relative to the first mountable body.

Optionally, the first mountable body is mountable to the at least one screw thread of the connecting means. Preferably, the first mountable body is mountable to the screw thread of the connecting means.

Optionally, the first mountable body is engagable with at least one of the respective ends of the expandable section. Further optionally, the first mountable body is irreversibly engagable with at least one of the respective ends of the expandable section.

Optionally, the first mountable body defines a portion of the expandable section. Further optionally, the first mountable body comprises a screw thread located on a portion of the expandable section.

Optionally, the first mountable body comprises a screw thread located on at least part of the inner surface of the expandable section.

Preferably, the first mountable body comprises a screw thread located on at least part of the inner surface of the expandable section.

Optionally, the screw thread located on at least part of the inner surface of the expandable section is mountable to the at least one screw thread of the connecting means. Preferably, the screw thread located on at least part of the inner surface of the expandable section is mountable to the screw thread of the connecting means.

Optionally or additionally, a second mountable body is fixedly mounted to the connecting means. Further optionally, the second mountable body is fixedly mounted to the connecting means to apply mechanical pressure to at least one respective end of the expandable section. The connecting means can be arranged to simultaneously and independently apply mechanical pressure to at least one respective end of the expandable section.

Optionally, the second mountable body is engagable with at least one of the respective ends of the expandable section. Further optionally, the second mountable body is reversibly engagable with at least one of the respective ends of the expandable section.

Optionally, the second mountable body defines a portion of the connecting means. Further optionally, the second mountable body comprises a stop located on a portion of the connecting means.

Optionally, the bolt further comprises at least one coupling.

According to a fifth aspect of the present invention there is provided a bolt for the fixation of bones, the bolt comprising: (a) a body having an expandable section and an outer surface, wherein at least a part of the outer surface of the body comprises at least one threaded portion; (b) expanding means; and (c) at least one coupling.

Preferably, the bolt comprises a coupling.

Optionally, the at least one coupling is mountable to the bolt.

Optionally, the at least one coupling is reversibly mountable to the bolt.

Optionally, the at least one coupling is fixedly mountable to the bolt.

Preferably, the at least one coupling is reversibly mountable to the bolt.

Optionally, the at least one coupling is mountable to the bolt by an interference fit.

Optionally, the at least one coupling is reversibly mountable to the bolt by an interference fit.

Preferably, the at least one coupling is reversibly mountable to the bolt by an interference fit.

Optionally, the bolt comprises means for mounting the at least one coupling to the bolt.

According to a sixth aspect of the present invention there is provided a bolt for the fixation of bones, the bolt comprising: (a) a body having an expandable section and an outer surface, wherein at least a part of the outer surface of the body comprises at least one threaded portion; (b) expanding means; (c) at least one coupling; and (d) mounting means.

Optionally, the bolt comprises means for reversibly mounting the at least one coupling to the bolt.

Optionally, the bolt comprises means for fixedly mounting the at least one coupling to the bolt.

Preferably, the bolt comprises means for reversibly mounting the at least one coupling to the bolt.

Optionally, the bolt comprises means for mounting the at least one coupling to the bolt by an interference fit.

Optionally, the bolt comprises means for reversibly mounting the at least one coupling to the bolt by an interference fit.

Preferably, the bolt comprises means for reversibly mounting the at least one coupling to the bolt by an interference fit.

Optionally, the body comprises the at least one coupling.

Optionally, the threaded portion comprises the at least one coupling.

Optionally, the body comprises the mounting means.

Optionally, the threaded portion comprises the mounting means.

Optionally, the body comprises the at least one coupling and the mounting means.

Optionally, the threaded portion comprises the at least one coupling and the mounting means.

Optionally, the proximal end of the body comprises the at least one coupling.

Optionally, the proximal end of the threaded portion comprises the at least one coupling.

Optionally, the proximal end of the body comprises the mounting means.

Optionally, the proximal end of the threaded portion comprises the mounting means.

Preferably, the proximal end of the threaded portion comprises the mounting means.

Optionally, the proximal end of the body comprises the at least one coupling and the mounting means.

Optionally, the proximal end of the threaded portion comprises the at least one coupling and the mounting means.

Optionally, the mounting means comprises a joint.

Optionally, the mounting means comprises a moveable joint.

Optionally, the mounting means comprises a pivotable joint.

Optionally, the mounting means comprises a ball joint.

Optionally, the mounting means comprises a ball-and-socket joint.

Preferably, the mounting means comprises a ball-and-socket joint.

Optionally, the proximal end of the body comprises the socket and the at least one coupling comprises the ball.

Optionally, the proximal end of the threaded portion comprises the socket and at least one coupling comprises the ball.

Optionally, the proximal end of the body comprises the ball and the at least one coupling comprises the socket.

Optionally, the proximal end of the threaded portion comprises the ball and at least one coupling comprises the socket.

Preferably, the proximal end of the body comprises the ball and the at least one coupling comprises the socket.

Preferably, the proximal end of the threaded portion comprises the ball and at least one coupling comprises the socket.

Optionally, the bolt further comprises linking means.

According to a seventh aspect of the present invention there is provided a bolt for the fixation of bones, the bolt comprising: (a) a body having an expandable section and an outer surface, wherein at least a part of the outer surface of the body comprises at least one threaded portion; (b) expanding means; (c) at least one coupling; (d) mounting means; and (e) linking means.

According to an eighth aspect of the present invention there is provided a system for the fixation of bones, the system comprising at least one bolt comprising: (a) a body having an expandable section and an outer surface, wherein at least a part of the outer surface of the body comprises at least one threaded portion; (b) expanding means; (c) at least one coupling; (d) mounting means; and (e) linking means.

Optionally, the system comprises (i) at least one bolt comprising: (a) a body having an expandable section and an outer surface, wherein at least a part of the outer surface of the body comprises at least one threaded portion; (b) expanding means; (c) at least one coupling; and (d) mounting means; and (ii) linking means.

Optionally, the system comprises at least two, optionally, at least three, optionally at least four bolts.

Optionally, the at least one coupling is mountable to the linking means.

Optionally, the at least one coupling is reversibly mountable to the linking means.

Preferably, the at least one coupling is reversibly mountable to the linking means.

Optionally, the at least one coupling is fixedly mountable to the linking means.

Optionally, the at least one coupling is arranged to receive the linking means.

Optionally, the at least one coupling is arranged to reversibly receive the linking means.

Optionally, the at least one coupling comprises at least one groove.

Optionally, the at least one coupling comprises at least one groove arranged to receive the linking means.

Optionally, the at least one coupling comprises at least one groove arranged to reversibly receive the linking means.

Optionally, the at least one coupling comprises at least one arm.

Optionally, the at least one coupling comprises at least two arms.

Preferably, the at least one coupling comprises two arms.

Optionally, the at least one coupling comprises at least two arms defining the groove therebetween.

Preferably, the at least one coupling comprises two arms defining the groove therebetween.

Optionally, the at least one coupling comprises at least two arms defining the groove arranged to reversibly receive linking means therebetween.

Preferably, the at least one coupling comprises two arms defining the groove arranged to reversibly receive linking means therebetween.

Optionally, the linking means comprises a rod.

Optionally, the linking means comprises a linear rod.

Optionally, the linking means comprises a non-linear rod.

Optionally, the linking means comprises a curved rod.

Optionally, the linking means comprises a cylindrical rod.

Optionally, the linking means comprises a linear cylindrical rod.

Optionally, the linking means comprises a non-linear cylindrical rod.

Optionally, the linking means comprises a curved cylindrical rod.

Optionally, the linking means comprises a circular cylindrical rod.

Optionally, the linking means comprises a linear circular cylindrical rod.

Optionally, the linking means comprises a non-linear circular cylindrical rod.

Optionally, the linking means comprises a curved circular cylindrical rod.

Optionally, the linking means comprises a rod having a non-circular cross-section.

Optionally, the linking means comprises a linear rod having a non-circular cross-section.

Optionally, the linking means comprises a non-linear rod having a non-circular cross-section.

Optionally, the linking means comprises a curved rod having a non-circular cross-section.

Optionally, the linking means comprises a rod having a circular cross-section.

Optionally, the linking means comprises a linear rod having a circular cross-section.

Optionally, the linking means comprises a non-linear rod having a circular cross-section.

Optionally, the linking means comprises a curved rod having a circular cross-section.

Optionally, the at least one coupling comprises at least one arm having a respective end.

Optionally, the at least one coupling comprises at least two arms, each arm having a respective end.

Preferably, the at least one coupling comprises two arms, each arm having a respective end.

Optionally, the at least one coupling comprises means for securing the linking means.

According to a ninth aspect of the present invention there is provided a bolt for the fixation of bones, the bolt comprising: (a) a body having an expandable section and an outer surface, wherein at least a part of the outer surface of the body comprises at least one threaded portion; (b) expanding means; (c) at least one coupling; (d) mounting means; (e) linking means; and (f) securing means.

According to a tenth aspect of the present invention there is provided a system for the fixation of bones, the system comprising at least one bolt comprising: (a) a body having an expandable section and an outer surface, wherein at least a part of the outer surface of the body comprises at least one threaded portion; (b) expanding means; (c) at least one coupling; (d) mounting means; (e) linking means; and (f) securing means.

Optionally, the system comprises (i) at least one bolt comprising: (a) a body having an expandable section and an outer surface, wherein at least a part of the outer surface of the body comprises at least one threaded portion; (b) expanding means; (c) at least one coupling; and (d) mounting means; (ii) linking means; and (iii) securing means.

Optionally, the securing means are mountable to the at least one coupling.

Optionally, the securing means are reversibly mountable to the at least one coupling.

Optionally, the securing means are fixedly mountable to the at least one coupling.

Preferably, the securing means are reversibly mountable to the at least one coupling.

Optionally, the securing means are mountable to the at least one arm having a respective end.

Optionally, the securing means are reversibly mountable to at least one arm having a respective end.

Optionally, the securing means are fixedly mountable to at least one arm having a respective end.

Optionally, the securing means are mountable to the at least two arms, each arm having a respective end.

Optionally, the securing means are reversibly mountable to the at least two arms, each arm having a respective end.

Optionally, the securing means are fixedly mountable to the at least two arms, each arm having a respective end.

Preferably, the securing means are reversibly mountable to the at least two arms, each arm having a respective end.

Optionally, the securing means comprises a set screw.

Optionally, the securing means comprises a set screw having at least one threaded portion.

Optionally, each arm comprises at least one threaded portion.

Optionally, each respective end of each arm comprises at least one threaded portion.

Optionally, each arm comprises at least one threaded portion corresponding to the at least one threaded portion of the set screw.

Optionally, each respective end of each arm comprises at least one threaded portion corresponding to the at least one threaded portion of the set screw.

Optionally, the securing means comprise means for rotating the set screw.

Optionally, the set screw comprises means for rotating the set screw.

Optionally, the securing means comprise means for rotating the set screw.

Optionally, the rotating means comprise a socket.

Optionally, the rotating means comprise a non-circular socket.

Optionally, the rotating means comprise a hexagonal socket.

Preferably, the rotating means comprise a hexagonal socket.

Optionally, the body has a proximal end having a proximal diameter and a distal end having a distal diameter, wherein the proximal diameter is greater than the distal diameter.

Optionally, the proximal diameter is the major diameter of the proximal end of the body.

Optionally, the distal diameter is the major diameter of the distal end of the body.

Optionally, the proximal diameter is the major diameter of the proximal end of the at least one treaded portion.

Optionally, the distal diameter is the major diameter of the distal end of the at least one treaded portion.

Optionally, the proximal diameter is the major diameter of the thread at the proximal end of the at least one treaded portion.

Optionally, the distal diameter is the major diameter of the thread at the distal end of the at least one treaded portion.

Optionally, the proximal diameter is the minor diameter of the proximal end of the body.

Optionally, the distal diameter is the minor diameter of the distal end of the body.

Optionally, the proximal diameter is the minor diameter of the proximal end of the at least one treaded portion.

Optionally, the distal diameter is the minor diameter of the distal end of the at least one treaded portion.

Optionally, the proximal diameter is the minor diameter of the thread at the proximal end of the at least one treaded portion.

Optionally, the distal diameter is the minor diameter of the thread at the distal end of the at least one treaded portion.

Optionally, the proximal diameter is at least 1.1 times greater than the distal diameter.

Optionally, the proximal diameter is at least 1.2, optionally at least 1.3, optionally at least 1.4, optionally at least 1.5, optionally at least 1.6, optionally at least 1.7, optionally at least 1.8, optionally at least 1.9, optionally at least 2.0 times greater than the distal diameter.

Preferably, the proximal diameter is at least 1.2 times greater than the distal diameter.

Optionally, the distal diameter is up to 6.5 mm.

Optionally, the distal diameter is up to 6.5, optionally up to 6.0, optionally up to 5.5, optionally up to 5.0, optionally up to 4.5, optionally up to 4.0, optionally up to 3.5, optionally up to 3.0, optionally up to 2.5, optionally up to 2.0, optionally up to 1.5, optionally up to 1.0, optionally up to 0.5 mm.

Preferably, the distal diameter is up to 6.5 mm.

Optionally, the proximal diameter is at least 1.0 mm.

Optionally, the proximal diameter is at least 1.5, optionally at least 2.5, optionally at least 3.0, optionally at least 3.5, optionally at least 4.0, optionally at least 4.5, optionally at least 5.0, optionally at least 5.5, optionally at least 6.0, optionally at least 6.5 mm.

Preferably, the proximal diameter is at least 6.5 mm.

Optionally, the proximal end and the distal end of the body form a non-linear slope therebetween.

Optionally, the proximal end and the distal end of the threaded portion form a non-linear slope therebetween.

Optionally, the proximal end and the distal end of the body form a linear slope therebetween.

Optionally, the proximal end and the distal end of the threaded portion form a linear slope therebetween.

Preferably, the proximal end and the distal end of the body form a linear slope therebetween.

Preferably, the proximal end and the distal end of the threaded portion form a linear slope therebetween.

Optionally, the thread of the at least one threaded portion is a straight thread.

Optionally, the thread of the at least one threaded portion is a tapered thread.

Optionally, the bolt further comprises at least one washer.

Optionally, the bolt comprises at least two washers.

Optionally, the bolt comprises two washers.

Optionally, the bolt comprises first and second washers.

Optionally, the at least one washer is substantially annular in shape. Further optionally, the at least one washer is substantially annular in shape and defines a washer aperture.

Optionally, the at least one washer is sized and dimensioned to receive the connecting means. Further optionally, the at least one washer is sized and dimensioned to receive the connecting means therethrough. Still further optionally, the at least one washer is sized and dimensioned to receive the connecting means through the washer aperture.

Optionally, the at least one washer is mountable to the connecting means. Further optionally, the at least one washer is reversibly mountable to the connecting means.

Optionally, the first washer is substantially annular in shape. Further optionally, the first washer is substantially annular in shape and defines a washer aperture.

Optionally, the first washer is sized and dimensioned to receive the connecting means. Further optionally, the first washer is sized and dimensioned to receive the connecting means therethrough.

Still further optionally, the first washer is sized and dimensioned to receive the connecting means through the washer aperture. Still further optionally, the first washer is sized and dimensioned to receive a distal end of the connecting means through the washer aperture.

Optionally, the first washer is mountable to the connecting means. Further optionally, the first washer is reversibly mountable to the connecting means. Still further optionally, the first washer is reversibly mountable to a distal end of the connecting means.

Optionally, the first washer is sized and dimensioned to be received by or at the proximal end of the body. Optionally, the first washer is sized and dimensioned to be received by or at the proximal end of the body or the threaded portion.

Optionally, the second washer is substantially annular in shape. Further optionally, the second washer is substantially annular in shape and defines a washer aperture.

Optionally, the second washer comprises at least two parts. Further optionally, the second washer comprises at least two contiguous parts. Still further optionally, the second washer comprises at least two, optionally contiguous, parts, which together are substantially annular in shape. Still further optionally, the second washer comprises at least two, optionally contiguous, parts, which together are substantially annular in shape and define a washer aperture.

Optionally, the second washer is sized and dimensioned to receive the connecting means. Further optionally, the second washer is sized and dimensioned to receive the connecting means therethrough. Still further optionally, the second washer is sized and dimensioned to receive the connecting means through the washer aperture. Still further optionally, the first washer is sized and dimensioned to receive a proximal end of the connecting means through the washer aperture.

Optionally, the second washer is mountable to the connecting means. Further optionally, the second washer is reversibly mountable to the connecting means. Still further optionally, the second washer is reversibly mountable to a proximal end of the connecting means. Still further optionally, the second washer is reversibly mountable adjacent a mountable body comprising a stop located on the outer surface of the connecting means. Still further optionally, the second washer is reversibly mountable between a mountable body comprising a stop located on the outer surface of the connecting means and a mounting means.

Preferably, the bolt is formed of a material that is suitable for sterilisation, so as to be provided in a sterile packaged state for use.

Preferably, the material is autoclavable.

Preferably, the material is surgical stainless steel, but it will be seen that any material that is suitable for sterilisation and can impart the required mechanical strength may be used.

Optionally, the material is an alloy. Further optionally, the material is titanium. Still further optionally, the material is a titanium alloy.

Any part of the bolt can be coated or partially coated with a coating, which can be a hydrophilic and/or lubricious coating.

Optionally, the coating is a polymeric coating.

Optionally, the coating is a polymeric coating comprising at least two monomers, each monomer comprising substituted or unsubstituted para-benzenediyl rings (-C6H4-) and 1 ,2-ethanediyl bridges (-CH2-CH2-). Further optionally, the coating is a polymeric coating comprising a plurality of monomers, each monomer comprising substituted or unsubstituted para-benzenediyl rings (-C6H4-) and 1,2-ethanediyl bridges (-CH2-CH2-).

Optionally, the coating comprises parylene. Further optionally, the coating comprises an unsubstituted parylene or a substituted parylene. Still further optionally, the coating comprises a chlorinated (optionally at least one, optionally at least two, chlorine-substituted) parylene, a fluorinated (optionally at least one, optionally at least two, optionally at least three, optionally at least four, fluorine-substituted) parylene, an alkyl-substituted (optionally at least one, optionally at least two, methyl-, optionally ethyl-, substituted) parylene, an amine (optionally at least one amine-, optionally methylene-, substituted) parylene, an ethinyl (optionally at least one ethinyl-substituted) parylene, and combinations and co-polymers each thereof.

Still further optionally, the coating comprises a parylene selected from parylene A, AM, AM-2, AF-4, C, D, E, F, M, N, VT-4, X, and combinations and co-polymers each thereof.

For the purposes of the present disclosure, a user is a person who will undertake the operation of the bolt during routine use. Usually, this will be a medical professional, where routine use includes fixation of a bone of a patient. When in use, the bolt is oriented so as to have a proximal end and a distal end relative to said user.

A patient is defined as a person on whom the bolt will be used during routine operation.

### Brief description of the drawings

Embodiments of the invention will now be described, by way of example, with reference to the accompanying drawings, in which:
**Figure 1** is a perspective view of a bolt having an expandable section in the contracted position;
**Figure 2** is a perspective view of a bolt having an expandable section in the expanded position;

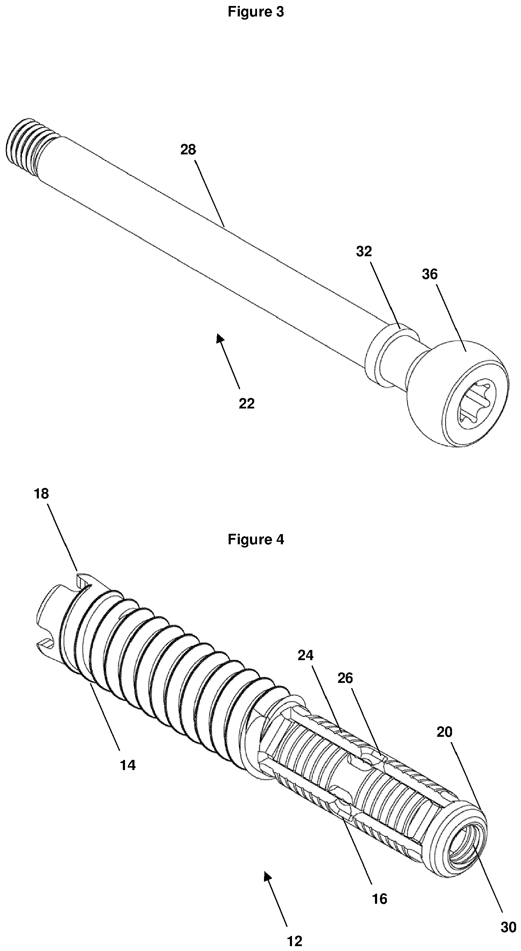

**Figure 3** is a perspective view of a connecting means of a bolt;
**Figure 4** is a perspective view of a body of a bolt;

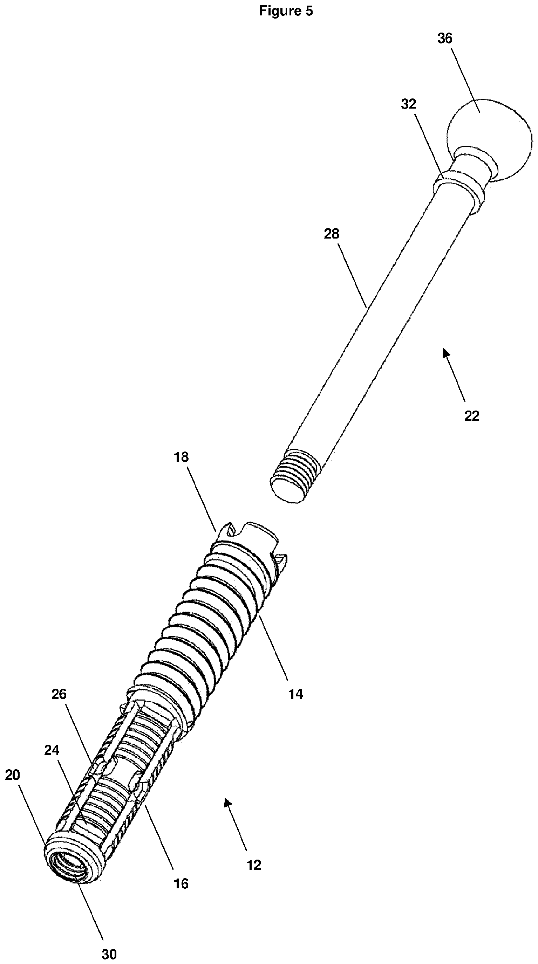

**Figure 5** is an exploded perspective view of a connecting means and a body of a bolt;

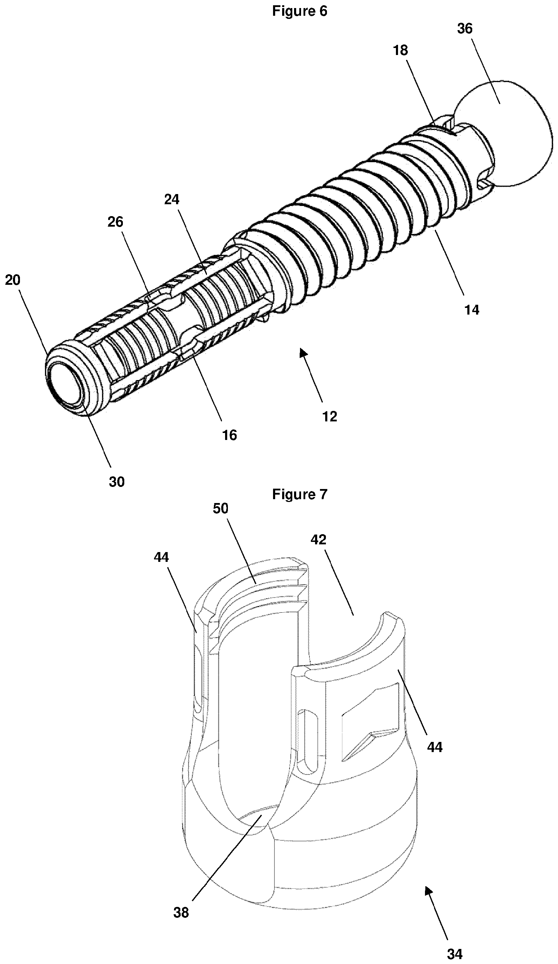

**Figure 6** is a perspective view of a connecting means and a body of a bolt;
**Figure 7** is a perspective view of a coupling of a bolt;

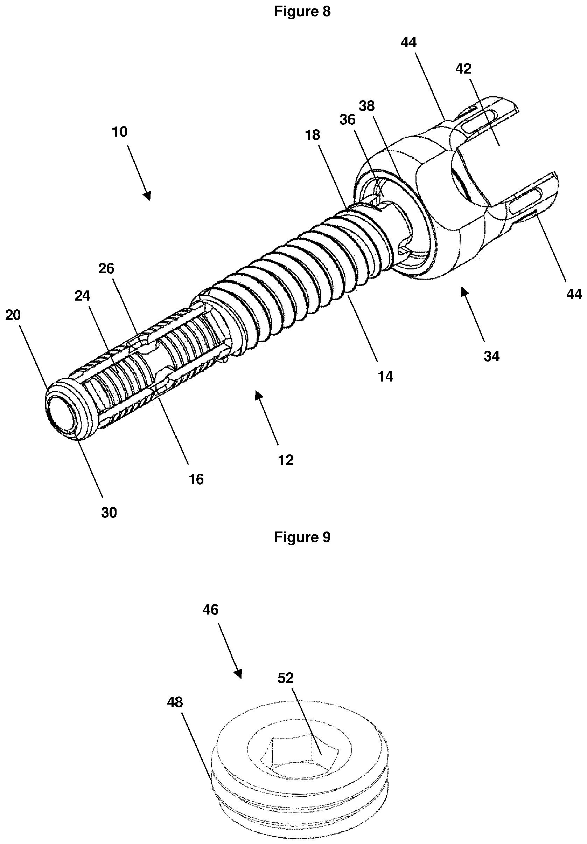

**Figure 8** is an exploded perspective view of a bolt having a coupling;
**Figure 9** is a perspective view of a securing means of a bolt;

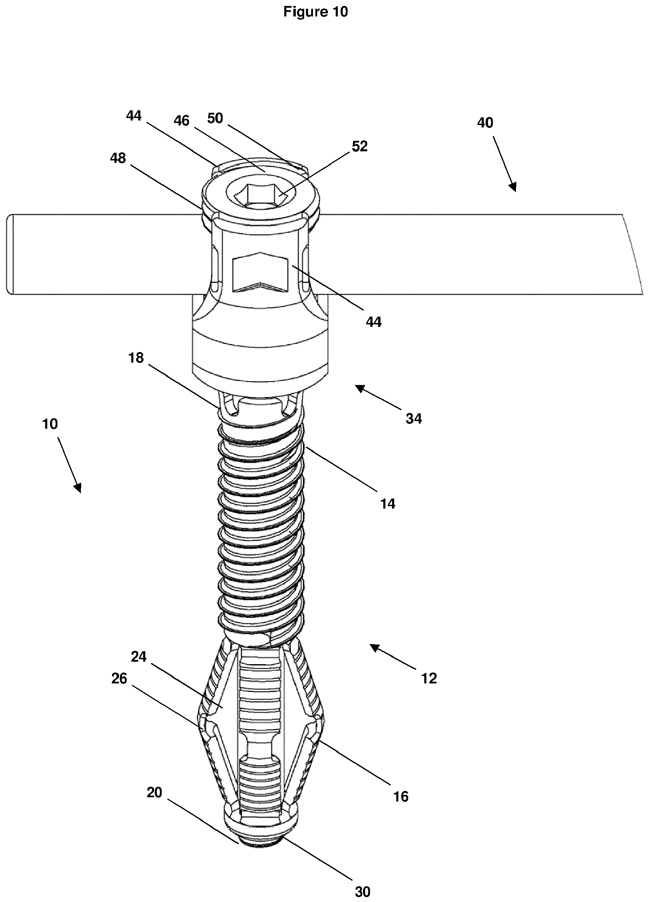

**Figure 10** is a perspective view of a system; and

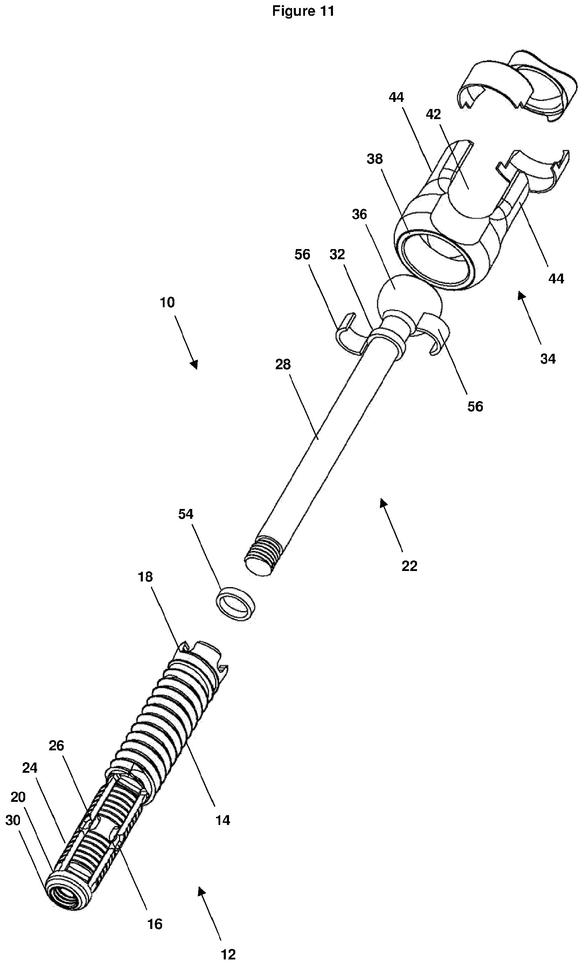

**Figure 11** is an exploded perspective view of a connecting means and a body of a bolt having at least one washer.

### Detailed description of the invention

The present invention relates to a bolt (10) for the fixation of bones.

In an embodiment, the bolt comprises a body (12) having an outer surface, wherein at least a part of the outer surface of the body comprises at least one threaded portion (14).

In an embodiment, the bolt comprising a body having an expandable section (16).

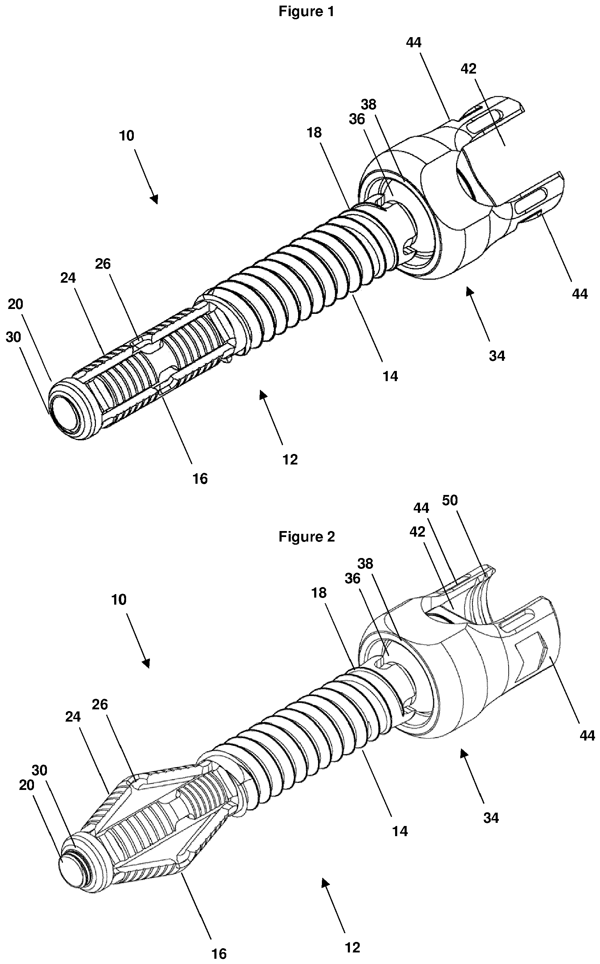

In a preferred embodiment, the bolt (10) comprises a body (12) having an expandable section (16) and an outer surface, wherein at least a part of the outer surface of the body comprises at least one threaded portion (14). The expandable section (16) is operable between a contracted position and an expanded position. The contracted position is illustrated in Figure 1 and the expanded position is illustrated in Figure 2.

In general, the expandable section (16) is adjacent the outer surface of the body (12). The expandable section (16) and the outer surface of the body (12) can be contiguous and/or conterminous and, in a preferred embodiment, the expandable section (16) and the outer surface of the body (12) are integral, being formed in one piece, although separate but adjoining pieces are possible. In use, the body (12) has a proximal end (18) and a distal end (20) and, generally, the expandable section (16) is adjacent or at the distal end (20) and the outer surface of the body (12) comprising the threaded portion (14) is adjacent or at the proximal end (18).

In an embodiment, the lead of the threaded portion (14) is greater than the pitch of the threaded portion (14). The lead of the threaded portion can be over two times, and up to over four times, greater than the pitch of the threaded portion (14) but, in a preferred embodiment, the lead of the threaded portion (14) is two times greater than the pitch of the threaded portion (14). The threaded portion (14) can comprise a thread having at least one lead (or a single-lead thread) or up to over four leads (including double-, triple-, or quadruple-lead threads) and, in a preferred embodiment, the threaded portion (14) comprises a thread having two leads (also known as a double-lead thread). The threaded portion (14) can comprise a thread having at least one start (or a single-start thread) or up to over four starts (including double-, triple-, or quadruple-start threads and, in preferred embodiment, the threaded portion (14) comprises a thread having two starts (also known as a double-start thread). The threaded portion (14) generally comprises a helical thread and, in some embodiments, can comprise up to over four helical threads but, in the preferred embodiment, the threaded portion (14) comprises two helical threads.

At least a part of the expandable section (16) can comprise a threaded portion, which can be the same or different to the threaded portion (14) of the outer surface of the body (12). In such an embodiment, the outer surface of the expandable section (16) comprises at least one threaded portion. However, in a preferred embodiment, the expandable section (16), or the outer surface thereof, does not comprise a threaded portion. In such an embodiment, the expandable section (16), or the outer surface thereof, excludes a threaded portion. Thus, the preferred embodiment of the bolt (10) comprises a body (12) having an expandable section (16), which excludes a threaded portion and an outer surface, which comprises a threaded portion (14).

The bolt (10) further comprises expanding means (22) in operable association with the expandable section (16) to displace the expandable section (16) between the contracted position and the expanded position by applying force to one or both of the respective ends of the expandable section (16), such that one or both of the respective ends of the expandable section (16) is advanced toward the opposing respective end. The expanding means (22) can displace the expandable section (16) between the contracted position and the expanded position by simultaneously applying force to the respective ends of the expandable section (16), whereby each of the respective ends of the expandable section (16) are advanced toward the opposing respective end.

In such a way, the expandable section (16) can be reversibly expandable, in some embodiments, reversibly expandable under mechanical pressure, such as the mechanical pressure applied by the expanding means (22) or the external force of the surrounding fixated bone. In such an embodiment, the expandable section (16) is collapsible, for example collapsible along a longitudinal axis, whereby the expandable section (16) can collapse radially inwardly to the contract position along a longitudinal axis.

The expandable section (16) comprises at least two expandable members (24) that extend from a longitudinal axis of the bolt (10) under mechanical pressure. In use, the expandable members (24) can extend radially from a longitudinal axis of the bolt (10) under mechanical pressure, such as the mechanical pressure applied by the expanding means (22). Each of the expandable members generally comprises a deformable arm. At least one point of folding (26) can be provided along each deformable arm. The point of folding can be a point of weakness, a hinge mechanism, or any such mechanism that will facilitate the folding of the deformable arm at a desired location.

The expanding means (22) generally comprises a connecting means (28) and at least one mountable body (30). The connecting means (28) comprises a shaft, which is preferably but not essentially adapted to allow reciprocal movement of the mountable body (30) relative to the connecting means (28) for example the shaft. The mountable body (30) can be threadably mountable to the connecting means (28) and can be arranged to simultaneously and independently apply mechanical pressure to one or both of the respective ends of the expandable section (16). In such an embodiment, the mountable body (30) can be displaced in response to rotation of the connecting means (28). For example, the connecting means (28) can comprise a screw thread, such that the mountable body (30) is mountable to the screw thread of the connecting means (28). In some embodiments, the mountable body (30) is engagable with a respective end of the expandable section (16) and can even be irreversibly engagable with a respective end of the expandable section (16). In a preferred embodiment, the mountable body (30) defines a portion of the expandable section (16), for example, the mountable body (30) can comprise a screw thread located on a portion of the expandable section (16) such a screw thread located on at least part of the inner surface of the expandable section (16). The screw thread located on the inner surface of the expandable section (16) is mountable to the screw thread of the connecting means (28).

Optionally or additionally, the mountable body can be fixedly mounted to the connecting means (28). For example, a second mountable body (32) is fixedly mounted to the connecting means (28) to apply mechanical pressure to a respective end of the expandable section (16). The connecting means (28) can be arranged to simultaneously and independently apply mechanical pressure to a respective end of the expandable section (16). This mountable body (32) can be engagable with a respective end of the expandable section (16) and is generally reversibly engagable with a respective end of the expandable section (16). In a preferred embodiment, this mountable body (32) defines a portion of the connecting means (28), for example, the mountable body (32) comprises a stop located on the outer surface of the connecting means (28).

In such a preferred embodiment, the expanding means (22) comprises a connecting means and two mountable bodies (30, 32) - first (30) and second (32) mountable bodies. The first mountable body (30) is threadably mountable to the connecting means (28), such that the first mountable body (30) can be displaced in response to rotation of the connecting means (28) relative to the first mountable body (30). In such an embodiment, the first mountable body (30) is mountable to the at least one screw thread of the connecting means (28). In this embodiment, the first mountable body (30) defines a portion of the expandable section (16) by comprising a screw thread located on the inner surface of the expandable section (16). The second mountable body (32) is fixedly mounted to the connecting means (28) and can define a portion of the connecting means (28) by comprising a stop located on a portion of the connecting means (28).

In a preferred embodiment, the bolt (10) further comprises a coupling (34), which can be mountable to the bolt (10) and which can be fixedly mountable to the bolt (10) but is preferably reversibly mountable to the bolt (10). The coupling (34) can be mountable to the bolt by, for example, an interference fit, thereby making the coupling (34) reversibly mountable to the bolt (10).

The bolt (10) can comprise means for mounting (36) the coupling (34) to the bolt (10). The bolt (10) can comprise means for reversibly mounting (36) the coupling (34) to the bolt (10) or for fixedly mounting the coupling (34) to the bolt (10). In a preferred embodiment, the bolt (10) comprises means for reversibly mounting (36) the coupling (34) to the bolt (10), for example, by an interference fit. In an embodiment, the body (12) comprises the coupling (34) and, in an embodiment, the threaded portion (14) comprises the coupling (34). Similarly, in an embodiment, the body (12) comprises the mounting means (36) and, in an embodiment, the threaded portion (14) comprises the mounting means (36). In this way, the proximal end (18) of the body (12) of the threaded portion (14) comprises the coupling (34). Similarly, the proximal end (18) of the body (12) of the threaded portion (14) comprises the mounting means (36).

The mounting means (36) can comprise a joint, which can be, for example, a moveable joint, such as a pivotable joint. In such an embodiment, the mounting means (36) comprises a ball joint and, preferably, the mounting means (36) comprises a ball-and-socket joint. Although the proximal end (18) of the body (12) or of the threaded portion (14) can comprise the socket and the coupling (34) can comprise the ball, in a preferred embodiment, the proximal end (18) comprises the socket (38) and the coupling (34) comprises the ball (36). In such an embodiment, the proximal end (18) of the body (12), and optionally of the threaded portion (14), comprises the ball (36) and the coupling (34) comprises the socket (38).

The bolt (10) can comprise linking means (40). In such a way, there is provided a system comprising linking means (4); and at least one bolt (10), each bolt (10) comprising a body (12) having an expandable section (16) and an outer surface, wherein at least a part of the outer surface of the body comprises at least one threaded portion (14); expanding means (26); a coupling (34); and mounting means (36). The system can comprise any number of bolts (10) including at least two, three, four, or more bolts (10).

The linking means (40) can comprises a rod, which can be a linear rod or a non-linear rod, such as a curved rod. The linking means (40) can comprises a cylindrical (for example circular cylindrical) rod, which can be a linear cylindrical rod or a non-linear or curved cylindrical rod. In some embodiments, the linking means (40) comprise a rod having a non-circular cross-section, which can be a linear or non-linear rod having a non-circular cross-section. However, in some embodiments, the linking means (40) comprises a rod having a circular cross-section, such as a linear or non-linear (such as curved) rod having a circular cross-section.

The coupling (34) is mountable to the linking means (40) and can be reversibly or fixedly mountable to the linking means (40), but is preferably reversibly mountable to the linking means (40). In this way, the coupling (34) is arranged to receive or reversibly receive the linking means (40). For example, the coupling (34) can comprise a groove (42), which can be arranged to receive or reversibly receive the linking means (40). In a preferred embodiment, the coupling (34) comprises two arms (44). The arms (44) can define the groove (42) between them, that is to say, the groove (44) can be defined between the arms (44). In this way, the two arms (44) defining the groove (42) are arranged to receive or reversibly receive the linking means (40) between them and within the groove (42).

The coupling (34) comprises means for securing (46) the linking means (40). The securing means (46) are mountable to the coupling (34) and can be reversibly or fixedly mountable to the coupling (34) but, is preferably reversibly mountable to the coupling (34). In a preferred embodiment, the securing means (46) are mountable to an or both arms (44), each of which has a respective end. The securing means (46) can comprise a set screw, which can have a threaded portion (48).

Correspondingly, each arm (44) comprises a threaded portion (50), preferably at each respective end of each arm (44). The threaded portion (50) of the arm (44) corresponds to the threaded portion (48) of the set screw (46). The securing means (46), for example the set screw, can comprise means for rotating (52) the securing means (46) (for example the set screw). The rotating means (52) can be a socket, which can be a non-circular socket, such as a hexagonal socket.

In an embodiment, the body (12) has a proximal end (18) having a proximal diameter and a distal end (20) having a distal diameter, wherein the proximal diameter is greater than the distal diameter. The proximal diameter can be the major diameter of the proximal end (18) of the body (12) or the treaded portion (14). The distal diameter can be the major diameter of the distal end (20) of the body (12) or the treaded portion (14). Alternatively, the proximal diameter is the minor diameter of the proximal end (18) of the body (12) or the treaded portion (14). The distal diameter can be the minor diameter of the distal end (20) of the body (12) or the treaded portion (14). The proximal diameter can be at least 1.1 times greater than the distal diameter, for example at least 1.2, at least 1.3, at least 1.4, at least 1.5, at least 1.6, at least 1.7, at least 1.8, at least 1.9, or at least 2.0 times greater than the distal diameter. In an embodiment, the distal diameter is up to 6.5 mm for example up to 6.5, up to 6.0, up to 5.5, up to 5.0, up to 4.5, up to 4.0, up to 3.5, up to 3.0, up to 2.5, up to 2.0, up to 1.5, up to 1.0, up to 0.5 mm. The proximal diameter can be at least 1.0 mm, for example at least 1.5, at least 2.5, at least 3.0, at least 3.5, at least 4.0, at least 4.5, at least 5.0, at least 5.5, at least 6.0, at least 6.5 mm.

The proximal end (18) and the distal end (20) of the body (12) or of the threaded portion (14) can form a non-linear or a linear slope therebetween but, preferably the proximal end (18) and the distal end (20) of the body (12) or the threaded portion (14) form a linear slope therebetween.

The thread of the threaded portion (14) can be a straight thread or a tapered thread.

In any of the embodiments described, the bolt (10) can further comprise at least one washer (54, 56). In some embodiments, the bolt comprises first (54) and second (56) washers. Each washer (54, 56) is substantially annular in shape and defines a washer aperture. Each washer (54, 56) is sized and dimensioned to receive the connecting means (28), such that the connecting means (28) can pass therethrough i.e. through the washer aperture. Each washer (54, 56) is mountable, optionally reversibly mountable, to the connecting means (28).

The first washer (54) can be substantially annular in shape and sized and dimensioned to receive a distal end of the connecting means (28) through the washer aperture. In such a way, the first washer (54) is reversibly mountable to the connecting means (28), preferably by a distal end of the connecting means (28). The first washer (54) can also be sized and dimensioned to be received by or at the proximal end (18) of the body (12) or of the threaded portion (14). Thus, the first washer (54) can be located, in use, by or at the proximal end (18) of the body (12) or of the threaded portion (14) and can receive a distal end of the connecting means (28) through the washer aperture.

The second washer (56) can be substantially annular in shape and define a washer aperture. In a preferred embodiment, the second washer (56) comprises two parts, which can be two contiguous parts, and which together are substantially annular in shape i.e. together form an annulus defining a washer aperture. The second washer (56) is mountable, optionally reversibly mountable, to a proximal end of the connecting means (28). Preferably, the second washer (56) is reversibly mountable adjacent a mountable body (32) comprising a stop located on the outer surface of the connecting means (28). The second washer (56) can be reversibly mountable between the mountable body (32) comprising the stop and the mounting means (36).

Each washer can be coated or partially coated with a coating, which can be a hydrophilic and/or lubricious coating in order to reduce frictional impedance or increase lubrication between any moving parts of the bolt.

## Claims

1. A bolt for the fixation of bones, the bolt comprising
(a) a body having
(i) an expandable section having respective ends and operable between a contracted position and an expanded position; and
(ii) an outer surface, wherein at least a part of the outer surface of the body comprises at least one threaded portion; and
(b) expanding means in operable association with the expandable section to displace the expandable section between the contracted position and the expanded position.

2. A bolt according to claim 1, wherein the expanding means comprises a connecting means and at least one body mountable to the connecting means, wherein the connecting means comprises a shaft adapted to allow reciprocal movement of the at least one mountable body relative to the connecting means.

3. A bolt according to claim 2, wherein a first mountable body is threadably mountable to the connecting means and a second mountable body is fixedly mounted to the connecting means.

4. A bolt according to claim 3, wherein the first mountable body comprises a screw thread located on at least part of the inner surface of the expandable section and which is mountable to a screw thread of the connecting means, and wherein the second mountable body comprises a stop located on a portion of the connecting means.

5. A bolt according to any one of claims 1-4, wherein the expandable section is reversibly expandable.

6. A bolt according to any one of claims 1-5 further comprising at least one coupling mountable to the bolt.

7. A bolt according to claim 6, wherein the bolt comprises a ball-and-socket joint for mounting the at least one coupling to the bolt, wherein the body comprises the ball and the at least one coupling comprises the socket.

8. A bolt according to claim 6 or 7 further comprising linking means, wherein the at least one coupling is mountable to the linking means, optionally wherein the linking means comprises a linear rod or non-linear (curved) rod.

9. A bolt according to claim 8, wherein at least one coupling comprises at least one groove arranged to receive the linking means.

10. A bolt according to claim 9, wherein the at least one coupling comprises at least two arms, each arm having a respective end, and defining the groove therebetween.

11. A bolt according to claim 10, wherein the at least one coupling comprises means for securing the linking means, wherein the securing means are mountable to the at least two arms, each arm having a respective end.

12. A bolt according to claim 12, wherein the securing means comprises a set screw having at least one threaded portion, and wherein each respective end of each arm comprises at least one threaded portion corresponding to the at least one threaded portion of the set screw.

13. A bolt according to any one of claims 2-12 further comprising at least one washer, which is substantially annular in shape, mountable to the connecting means, and sized and dimensioned to be received by or at the proximal end of the body or the threaded portion.

14. A bolt according to any one of claims 1-13, wherein at least part of the bolt is coated or at least partially coated with a hydrophilic and/or lubricious coating.

15. A system for the fixation of bones, the system comprising at least one bolt comprising (a) a body having (i) an expandable section having respective ends and operable between a contracted position and an expanded position; and (ii) an outer surface, wherein at least a part of the outer surface of the body comprises at least one threaded portion; (b) expanding means in operable association with the expandable section to displace the expandable section between the contracted position and the expanded position, (c) at least one coupling; (d) mounting means; (e) linking means; and (f) securing means.
